(19) Europäisches Patentamt / European Patent Office / Office européen des brevets

(11) **EP 4 516 296 A1**

(12) **EUROPEAN PATENT APPLICATION**

(43) Date of publication:
**05.03.2025 Bulletin 2025/10**

(21) Application number: **24197910.3**

(22) Date of filing: **02.09.2024**

(51) International Patent Classification (IPC):
**A61K 9/51** (2006.01)   **A61K 9/107** (2006.01)
**A61K 47/10** (2017.01)   **A61K 47/36** (2006.01)

(52) Cooperative Patent Classification (CPC):
**A61K 9/0056; A61K 9/1075; A61K 47/10; A61K 47/36**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.08.2023 IT 202300017910**

(71) Applicant: **Laboratorio della Farmacia S.p.A.**
**30020 Quarto d'Altino VE (IT)**

(72) Inventor: **Fratter, Andrea**
**30030 Olmo di Martellago VE (IT)**

(74) Representative: **Perani & Partners S.p.A.**
**Piazza Armando Diaz, 7**
**20123 Milano (IT)**

(54) **NANOEMULSIFYING COMPOSITION CARRYING AT LEAST ONE VITAMIN ACTIVE INGREDIENT**

(57) The present invention relates to a nanoemulsifying composition comprising a carrier comprising or consisting of i) an oil phase containing at least one polyoxyethylene sorbitan ester, a medium chain triglyceride; ii) an aqueous phase containing a modified alkyl carboxylate starch, water; an active ingredient, dissolved or dispersed in the aqueous phase and/or in the oil phase, said active ingredient consisting of a vitamin selected from the group consisting of Vitamin $D_3$, Vitamin $K_2$, Vitamin $B_{12}$, and mixtures thereof.

Figure 2

**Description**

**FIELD OF THE INVENTION**

The invention is in the technical field of nanoemulsions, as a composition carrying active ingredients with low bioavailability.

**BACKGROUND ART**

The bioavailability of bioactive molecules in the pharmaceutical and nutraceutical sphere is a prerequisite for ensuring their efficacy. Precisely for this reason, lipophilic molecules or those that are poorly assimilated by the gut for various reasons should be administered with delivery technologies that are highly bioaccessible and/or capable of limiting massive pre-systemic biotransformation in the intestine and liver.

In particular, in the nutraceutical sphere, Vitamin $D_3$, Vitamin $K_2$ and Vitamin $B_{12}$ represent, in terms of diffusion, physiological importance and absorption problems, the main substances of which deficiency is widespread, especially in the mature and senile age.

Vitamin $D_3$ plays a key physiological role in promoting the absorption of calcium in the small intestine, thereby promoting the maintenance and trophism of bone structure. Vitamin $D_3$ also exerts an immunostimulating and anti-inflammatory action, modulating the release of various pro-inflammatory cytokines, such as IL-1, IL-6, IL-17, TNF-$\alpha$, especially during infectious diseases of the respiratory and rheumatic tracts [1-3].

Vitamin $K_2$ plays a physiological role in the coagulation cascade, activating prothrombin; on the other hand, it also plays a physiological role in inhibiting bone resorption and maintaining bone trophism, in association with Vitamin $D_3$ [4,5]. There is also published evidence about the protective action of vitamin K on the cardiovascular system [6].

Vitamin $B_{12}$, in its cyanocobalamin, methylcobalamin and hydroxocobalamin forms, has a fundamental function in maintaining red blood cell morphology and nerve trophism. It promotes correct nucleic acid synthesis in synergy with folic acid. Its deficiency causes macrocytic megaloblastic anaemia and even severe forms of neuritis.

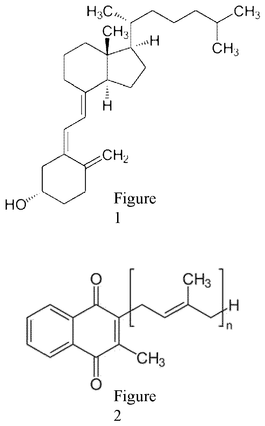

With regard to Vitamins $D_3$ and $K_2$, their secosteroid and isoprenoid quinone molecular structure respectively (Figures 1 and 2) makes them particularly lipophilic and poorly soluble in aqueous gastro-enteric fluids. Despite the micellar and emulsifying action exerted by the bile salts secreted by the liver into the duodenum, these substances are not always properly solubilised or dispersed; therefore, they are not uniformly assimilated by the enterocytes [7,8]. In addition to this, advancing age is associated with the progressive reduction of the biliary secretory function and the absorptive function of the intestine, thus creating conditions for poor assimilation.

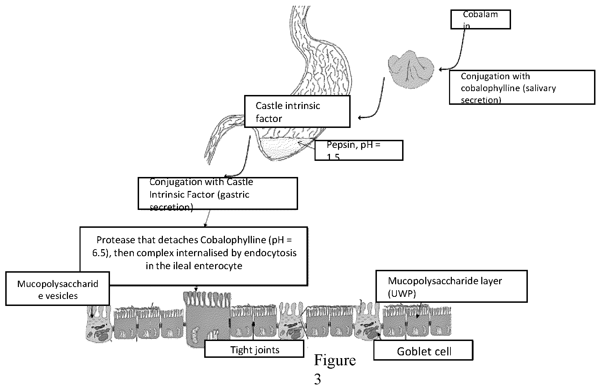

As far as Vitamin $B_{12}$ is concerned, its assimilation is physiologically driven by two fundamental steps: conjugation with haptocorrin, a protein secreted in saliva, which can protect it from gastric juices and promote its subsequent conjugation with Castle Intrinsic Factor (IF), secreted by the gastric parietal cells, which is crucial for the subsequent internalisation of Vitamin $B_{12}$ in the ileal enterocytes (Figure 3). For the same reasons already mentioned above, in old age, the assimilation of Vitamin $B_{12}$ may be impaired by reduced secretion of IF and poor receptivity of the intestinal mucosa. These phenomena are added to the reduced intake of Vitamin $B_{12}$ in the diet and the chronic use of drugs, such as Proton Pump Inhibitors (PPIs), which reduce or even eliminate the secretion of IF from the cells of the gastric mucosa.

In the Italian and European pharmaceutical and nutraceutical market, these substances are generally offered in oily formulations or in tablets or capsules.

In particular, Vitamin $D_3$ is widely prescribed and used in the form of an oil dispersion with vegetable oils or medium-chain triglycerides. The intake of these oily forms of vitamin $D_3$ is preferable with meals in order to evoke biliary secretion. However, recent clinical studies have shown that Vitamin $D_3$ in the form of sublingual nanoemulsion sprays is assimilated in a very effective and far superior way with respect to normal oily forms, especially in patients suffering from enteric malabsorption syndrome [9]. This evidence paves the way for the sublingual route of administration of Vitamin $D_3$ which, especially in individuals with dysphagia, xerostomia or other swallowing difficulties, may also represent a viable alternative to oral oily or solid forms.

The same considerations apply, albeit with the necessary differences in terms of delivery and diffusion, to vitamin $K_2$.

Vitamin $B_{12}$ is a water-soluble substance with a high molecular size, thus not suitable for sublingual permeation. However, recent work has demonstrated efficient sublingual permeation of Vitamin $B_{12}$, capable of developing a bioavailability comparable with the oral and even parenteral form [10,11]. Nanoemulsion may therefore represent a viable form of Vitamin $B_{12}$ assimilation through the lingual mucosa, due to its intrinsic characteristics as an absorption promoter. In this case, the presence of emulsifiers with the characteristics of "mucosal absorption enhancers" may increase trans-epithelial flux, promoting the bioavailability thereof.

*Problems of the prior art*

Nanoemulsions are transparent, liquid, clear or translucent systems that can promote a significant increase in enteric bioaccessibility and trans-mucosal bioavailability, but are not very or not at all chemically and/or physically stable, in the sense that they tend to cloud and/or lose their original dimensional characteristics over time.

In the food sector, where the use of emulsifiers is much more restrictive than in the pharmaceutical sector, obtaining stable nanoemulsions capable of guaranteeing the initial performance in conveying the active ingredients over time is particularly complex.

Specifically, the authors of WO2011073726A1 developed a food-grade nanoemulsion whose emulsifying system is essentially based on the following pair of emulsifiers: on the one hand, polysorbate 80, a non-ionic, high HLB emulsifier able to act as a solubiliser and O/W emulsifier; on the other hand, ascorbyl palmitate, a substance known and widely used as an antioxidant and anti-peroxidant in food, cosmetics and pharmaceuticals, but which also exerts an emulsifying action by virtue of its amphiphilic structure. This nanoemulsion has been described for delivering Vitamin $D_3$, melatonin and astaxanthin in the form of a sublingual spray [12,13].

Despite its good performance as a sublingual delivery system, this system demonstrates reduced stability due to the phenomenon of dimensional increase of the dispersed phase droplets over time (visible through the phenomenon of opacification, which is proportional to the increase in the average diameters of the dispersed phase droplets), and due to the instability of the ascorbyl palmitate, which, as it oxidises, leads to the darkening of the delivery system and the unpleasant taste given to the composition. Furthermore, for the stability and effectiveness of the emulsifying system, the authors of WO2011073726A1 consider the presence of an alkalising agent essential.

In the light of the already known nanoemulsions, carrying at least Vitamin $D_3$, Vitamin $K_2$ and/or Vitamin $B_{12}$, there is a need to develop innovative delivery systems in the form of nano-emulsifiers that overall:

-    are more stable in chemical and/or physical terms;

-    remain transparent over time, e.g. by avoiding browning;

-    are easily obtainable, in the sense that they require simple, low cost methods of preparation and easy application in industrial processes.

**SUMMARY OF THE INVENTION**

The Applicant has developed a nanoemulsifying composition (or nanoemulsion) comprising

a carrier comprising or consisting of

   i) an oil phase containing

-    at least one polyoxyethylene sorbitan ester,

-    a medium-chain triglyceride,

   ii) an aqueous phase containing

-    a modified alkyl carboxylate starch,

-    water,

an active ingredient, dissolved or dispersed in the aqueous phase and/or in the oil phase, said active ingredient consisting of a vitamin selected from the group consisting of Vitamin $D_3$, Vitamin $K_2$, Vitamin $B_{12}$, and mixtures thereof,

wherein the composition does not comprise ascorbic acid and/or ascorbyl-conjugated fatty acids, preferably it does not comprise ascorbyl palmitate.

A further object of the present invention is a process for preparing the above-mentioned nanoemulsion.

*Advantages of the invention*

The Applicant has developed a nanoemulsifying composition whose oil phase comprises at least one polyoxyethylene sorbitan ester and one medium-chain triglyceride, and whose aqueous phase comprises a modified alkyl carboxylate starch, as well as water. This innovative emulsifying system overcomes the aforementioned problems in the state of the art, resulting in a nanoemulsifying composition:

- which is stable over time in chemical and/or physical terms. In particular, a reduction or zeroing of the phenomenon of size increase of dispersed phase particles or droplets is observed;

- which is transparent and translucent over time; moreover, no undissolved aggregates are observed;

- in which no browning or oxidation phenomena are observed; no organoleptic changes are observed over time, unlike systems in which at least one emulsifying agent is ascorbic acid and/or ascorbyl-conjugated fatty acids. For these systems, after 30 days at room temperature, browning can be observed, a phenomenon related to the oxidation of ascorbate/yl by oxygen dispersed in water with generation of the dihydro form that induces colouration;

- which is easily obtainable, in the sense that it requires simple, low cost preparation methods, and easy application in industrial processes;

- which can be used in the pharmaceutical and/or food industry.

## DESCRIPTION OF THE DRAWINGS

*Figure 1* - Molecular structure of Vitamin $D_3$;
*Figure 2* - Molecular structure of Vitamin $K_2$;
*Figure 3* - Main stages of the Vitamin $B_{12}$ assimilation mechanism;
*Figure 4* - Comparison photo of the comparative laboratory test of Example 5;

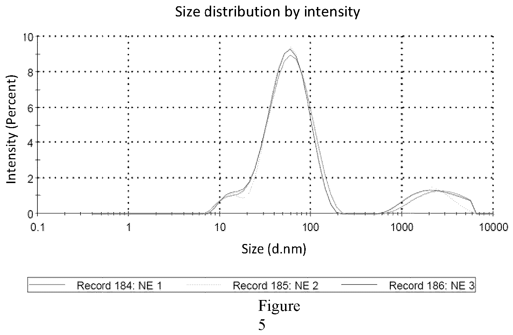

*Figure 5* - DLS analysis result of Example 6.

## DETAILED DESCRIPTION OF THE INVENTION

### *Nanoemulsifying composition (or nanoemulsion)*

In general, nanoemulsions are dispersed biphasic systems, in which a dispersed phase and a dispersing phase are observed, and in which the dispersed phase particles or droplets are characterised by a nanometric mean diameter.
Preferably, the nanoemulsifying composition comprises dispersed phase particles having an average diameter size comprised between 50 and 200 nanometres (*nm*), preferably between 50 and 150 nm, preferably between 50 and 100 nm, preferably between 60 and 100 nm, preferably between 70 and 100 nm, preferably between 80 and 100 nm.
The dispersed phase can be water or a fatty or oily phase; the dispersing phase is aqueous if the dispersed phase is fatty or oily, and vice versa. If the dispersed phase consists of an oil or fat phase and the dispersing phase consists of an aqueous phase, the nanoemulsion is called "oil-in-water" (O/W).
Nanoemulsions are transparent, clear, translucent systems with a slight bluish tint (*Bluish Tindall*), as the average size of the dispersed phase droplets is less than or equal to a quarter of the wavelength of the visible spectrum (about 100 nm).
Preferably, the nanoemulsifying composition of the invention is an oil-in-water nanoemulsion.
Preferably, the nanoemulsifying composition of the invention is a *liquid* (atomisable) composition at room temperature.
For the purposes of the present invention, room temperature means a temperature comprised between 23°C and 30°C, preferably between 23°C and 28°C, preferably between 23°C and 27°C, preferably between 23°C and 25°C.
The chemical and physical stability of a nanoemulsifying system also depends on the size of the droplets or particles of dispersed phase.
In particular, in order to reduce the size of the dispersed phase droplets, relatively large amounts of energy must be applied to overcome the interfacial tension $\gamma$ opposing them, in accordance with La Place's law (1) and Gibbs' law (2) below.

$$(1) \; \Delta P = \frac{2\gamma}{R} \, ,$$

wherein: $\Delta$P is the pressure difference between the inside $P_i$ and outside $P_e$ of the dispersed droplet, which is spherical on average. In particular, with $P_i > P_e$. $\gamma$ is the interfacial tension; $R$ is the radius of the droplet.

$$(2) \; \Delta G = \Delta H - (T \times \Delta S) \, ,$$

wherein: $\Delta G$ is the variation of Free Energy G, which is a measure of the spontaneity of a physical phenomenon; $\Delta H$ is the variation of Enthalpy $H$ of the physical system; T is the absolute temperature; $\Delta S$ is the variation of Entropy $S$ of the physical system.

It is possible to express the change in Enthalpy $\Delta H$ in the form of the following product ($\gamma \times Sup$), where Sup is the Surface Area of the dispersed phase spherical droplet, resulting in equation (3) below:

$$(3) \; \Delta G = (\gamma \times Sup) - (T \times \Delta S)$$

From equation (1), it can therefore be deduced that, if, as $R$ is reduced, $\gamma$ can be proportionally reduced, it becomes easier to overcome the internal pressure $P_i$ of the dispersed phase droplet, thereby reducing its size. In this sense, it is necessary to introduce a suitable emulsifying system to reduce $\gamma$, so as to facilitate the reduction of the average diameter of the dispersed phase droplets.

From equation (3), it can also be derived, that as $\gamma$ tends to values close to zero or at any rate very small, the product ($T \times \Delta S$), an indicator of the chaotic state of the system generated by the migration of a very large number of droplets into the external dispersing phase, will end up prevailing in absolute value over $\Delta H$. In other words, as the water/oil interfacial tension decreases, it is easier to reduce the size of the dispersed phase droplets. Having reached a certain dimensional reduction, the system is characterised by such a large number of dispersed phase droplets that it generates chaotic motion capable of increasing the entropy of the system. Hence, the product ($T \times \Delta S$) exceeds in absolute value that of the enthalpy $\Delta H$, thus making the formation process of the microemulsion spontaneous from an energetic point of view. In this case, we speak of microemulsions (mesotropic or bicontinuous systems), i.e. systems which are both kinetically and thermodynamically stable over time, unlike nanoemulsions which are only kinetically stable.

In general, another way to reduce the size of the droplets is to apply a large amount of energy mechanically, e.g. by using high-pressure homogenisers.

In the following, the percentage quantity of the components is expressed as a percentage of the weight of the component with respect to the total volume of the composition (% w/V).

### *Active ingredients*

Preferably, the composition constitutes a carrier for an active ingredient consisting of a vitamin, in the sense that the active ingredient is dispersed or soluble in an oil and/or aqueous phase, depending on its chemical nature.

In other words, the nanoemulsion of the invention constitutes a time-stable delivery system (or carrier) for the active ingredients included in the composition.

The vitamin contained in the nanoemulsion is chosen from the group consisting of: Vitamin $D_3$, Vitamin $K_2$, Vitamin $B_{12}$, and mixtures thereof.

Preferably, the aforementioned vitamin constitutes the *only* active ingredient included in the nanoemulsifying composition of the invention.

Preferably, Vitamin $D_3$ is in an amount comprised between 0.005% and 0.02% by weight, preferably 0.009% and 0.02% by weight, preferably 0.01% and 0.02% by weight, preferably 0.016% by weight, of the total volume of the composition.

Preferably, the nanoemulsifying composition is liquid and/or in nebulisable form and, for a 0.2 ml administration, Vitamin $D_3$ is in a variable amount such that a minimum of 12.5 $\mu$g (or mcg) (500 IU) and a maximum of 50 $\mu$g (2000 IU) are guaranteed.

In pharmacological terms, an International Unit (IU) is a unit of measurement of the quantity of a substance based on its biological effect or activity.

Preferably, Vitamin $K_2$ is in an amount comprised between 0.02% and 0.035% by weight, preferably between 0.025% and 0.03% by weight, preferably between 0.027% and 0.03% by weight, preferably about 0.027% by weight, of the total volume of the composition.

Preferably, the nanoemulsifying composition is liquid and/or in nebulisable form and, for a 0.2 ml administration, Vitamin $K_2$ is in a variable amount such that a minimum of 25 $\mu$g and a maximum of 100 $\mu$g are guaranteed.

Preferably, Vitamin $B_{12}$ is in an amount comprised between 0.1% and 0.5% by weight, preferably between 0.2% and 0.4% by weight, preferably about 0.3% by weight, of the total volume of the composition.

Preferably, the nanoemulsifying composition is liquid and/or in nebulisable form and, for a 0.2 ml administration, Vitamin $B_{12}$ is in a variable amount such that a minimum of 2 μg and a maximum of 50 μg are guaranteed.

Preferably, the nanoemulsifying composition comprises Vitamin $D_3$, Vitamin $K_2$ and Vitamin $B_{12}$ in a total amount comprised between 0.015% and 0.4% by weight, preferably between 0.016% and 0.37% by weight, preferably between 0.016% to 0.36% by weight, preferably between 0.02% to 0.36% by weight, preferably between 0.1% to 0.36% by weight, preferably between 0.2% to 0.36% by weight, preferably between 0.27% to 0.36% by weight, preferably between 0.33% to 0.36% by weight, of the total weight of the composition.

*Oil phase*

The nanoemulsifying composition comprises an oil phase (or fat phase), which preferably constitutes the dispersed phase, and which contains or consists of:

- at least one polyoxyethylene sorbitan ester, preferably *one* polyoxyethylene sorbitan ester;

- a medium-chain triglyceride;

- optionally suitable excipients.

In cases where the vitamins being delivered are vitamin $K_2$ and/or vitamin $D_3$, these active ingredients will preferably be dispersed/dissolved in the fat phase of the nanoemulsion.

Preferably, the at least one polyoxyethylene sorbitan ester is selected from the group consisting of: polysorbate 20, polysorbate 40, polysorbate 60, polysorbate 65, polysorbate 80, and mixtures of the foregoing; preferably, the at least one polyoxyethylene sorbitan ester is selected from polysorbate 20 and polysorbate 80.

According to a preferred form, the at least one polyoxyethylene sorbitan ester is polysorbate 80, for industrial operation reasons. Polysorbate 80 is in liquid form.

Preferably, the at least one polyoxyethylene sorbitan ester is in an amount comprised between 0.3% and 10% by weight, preferably between 0.5% and 10% by weight, preferably between 0.7% and 10% by weight, preferably between 0.9% and 10% by weight, preferably between 0.98% and 10% by weight, preferably between 1% and 10% by weight, preferably between 2% and 10% by weight, preferably between 2% and 8% by weight, preferably between 2% and 7% by weight, preferably between 3% and 6% by weight, preferably between 3.9% and 5.8% by weight, of the total volume of the composition.

Alternatively, the at least one polyoxyethylene sorbitan ester is comprised between 0.9% and 6%, preferably 0.98% by weight, or 3.9% by weight, or 5.8% by weight, of the total volume of the composition.

Preferably, the medium chain triglyceride (MCT) is a triglyceride of saturated fatty acids extracted from vegetable oils, preferably extracted from coconut oil and/or palm oil; preferably, it is a triglyceride of saturated fatty acids, in which the number of carbon atoms of the fatty acids is comprised between C8 and C10.

Preferably, the medium-chain triglyceride is the triglyceride of caprylic (C8) and capric (C10) fatty acids (triglyceride INCI name: *Caprylic/Capric Triglyceride*).

Please note that commercially available ingredients classifiable as "triglycerides of fatty acids" possibly contain fractions or traces of *mono* and *di*glycerides of fatty acids, preferably in a negligible amount by weight of the total weight; preferably, any *mono* and *di*glycerides of fatty acids are in an amount < 1% by weight of the total weight of the commercial product.

Preferably, the medium-chain triglyceride is in an amount comprised between 0.5% and 5% by weight, preferably between 0.8% and 5% by weight, preferably between 0.8% and 3% by weight, preferably equal to 0.8% by weight, or approximately 2.7% by weight, or 3% by weight, of the total volume of the composition.

*Aqueous phase*

The nanoemulsifying composition comprises an aqueous phase, which preferably constitutes the dispersing phase, containing or consisting of

- a modified alkyl carboxylate starch,

- water,

- optionally, suitable excipients and/or diluents.

In the case in which the vitamin being delivered is vitamin $B_{12}$, this active ingredient is preferably dispersed/dissolved in the aqueous phase of the nanoemulsion.

Modified alkyl carboxylate starch means chemically modified starch, i.e. starch contained in cereals and tubers which has undergone a chemical esterification treatment with an alkyl carboxylic acid, wherein the carboxylic acid is preferably an organic acid with a number of carbon atoms comprised between C2 and C5, preferably it is C4, and wherein the alkyl of the carboxylic acid preferably has a number of carbon atoms comprised between C3 and C10, preferably between C5 and C8, preferably equal to C8. The esterification reaction occurs between the carboxylic acid group (-COOH), with the alcohol group (-OH) of the glucose units of the starch.

Preferably, the modified alkyl carboxylate starch is selected from: sodium starch octenyl succinate (or starch sodium octenyl succinate, E1450) and aluminate starch octenyl succinate (starch aluminium octenyl succinate, E1452).

Note that sodium starch octenyl succinate is a natural, chemically modified starch produced by esterification of food starch (from rice or maize) with octenylsuccinic anhydride, using sodium carbonate or sodium hydroxide as a pH buffer.

Note that aluminium starch octenyl succinate is starch esterified with octenylsuccinic anhydride and treated with aluminium sulphate.

It should be noted that modified alkyl carboxylate starch, preferably sodium starch octenylsuccinate and/or aluminated starch octenylsuccinate, does not possess

*mucosal absorption enhancer* properties. An *"absorption enhancer"* refers to an agent capable of increasing the absorption of an active by enhancing its permeation in the mucous membranes.

Preferably, the modified alkyl carboxylate starch is in an amount comprised between 0.3% and 5% by weight, preferably between 0.5% and 5% by weight, preferably between 0.7% and 5% by weight, preferably between 0.9% and 5% by weight, preferably between 0.9% and 3% by weight, preferably between 0.9% and 2% by weight, preferably 0.98% by weight, or 1.96% by weight, of the total volume of the composition.

Preferably, the water is in an amount comprised between 75% and 95% by weight, preferably between 75% and 93% by weight, preferably between 80% and 93% by weight, preferably between 82% and 93% by weight, preferably between 85% and 93% by weight, preferably between 86% and 90% by weight, preferably 81.4% by weight, or about 83% by weight, or about 86% by weight, or about 89.5% by weight, of the total volume of the composition.

It should be noted that the nanoemulsifying composition does not comprise ascorbic acid and/or ascorbyl-conjugated fatty acids, preferably the nanoemulsifying composition does not comprise ascorbyl palmitate.

Ascorbyl-conjugated fatty acids are defined as fatty acids characterised by a number of carbon atoms comprised between C10 and C18, preferably comprised between C12 and C16, preferably C16, which are covalently bonded to the ascorbic acid. Preferably, the ascorbyl-conjugated fatty acid is ascorbyl palmitate.

It should be noted that the nanoemulsifying composition exhibits chemical and physical stability although it does not comprise ascorbic acid and/or ascorbyl-conjugated fatty acids. The absence of ascorbic acid and/or ascorbyl-conjugated fatty acids guarantees the transparency of the nanoemulsifying composition over time, as well as the absence of browning, oxidation or organoleptic changes over time.

Preferably, the nanoemulsifying composition is chemically and physically stable over a time period of $\geq 30$ days, preferably $\geq 1$ month, preferably between 30 and 60 days, at a temperature comprised between 35°C and 45°C, preferably 40°C.

Preferably, the nanoemulsifying composition is chemically and physically stable for $\geq 6$ months, preferably comprised between 6 and 12 months, preferably 12 months, at room temperature.

According to a preferred embodiment, the nanoemulsifying composition contains as a *single emulsifying agent* the emulsifying pair consisting of the at least one polyoxyethylene sorbitan ester, for example polysorbate 80, and the modified alkyl carboxylate starch, preferably sodium octenylsuccinate starch.

Preferably, the Applicant considers that the aforementioned effects may result not only from the qualitative choice of the emulsifying pair, but also from the weight ratio between the amount of the modified alkyl carboxylate starch and the amount of the at least one polyoxyethylene sorbitan ester; preferably, the weight ratio between the amount of the modified alkyl carboxylate starch and the amount of the at least one polyoxyethylene sorbitan ester is comprised between 1:1 to 1:6, preferably between 1:1 and 1:5, preferably between 1:1 and 1:4, preferably 1:1, or 1:3 or 1:4.

Therefore, the Applicant considers that, in order to obtain a transparent and stable nanoemulsion over time, the presence of the emulsifying pair of at least one polyoxyethylene sorbitan ester and modified alkyl carboxylate starch is essential. The mere presence of the modified alkyl carboxylate starch is not sufficient for the above-mentioned purposes (see Example 5).

According to a preferred form, the nanoemulsifying composition does not comprise an alkalising agent. An alkalinising agent is defined as a substance capable of alkalinising a dispersion or solution to a pH $\geq 6$ or 7.

According to a particularly preferred form, the nanoemulsifying composition does not comprise an alkalinising agent selected from: arginine, lysine, peptide, aminomethylpropanol, alkaline metal hydroxide, alkaline earth metal hydroxide, and mixtures thereof.

The alkalising agent is known to be used, for example, to ensure emulsifying efficacy in emulsifying systems based on ascorbic acid and/or ascorbyl-conjugated fatty acids, such as ascorbyl palmitate.

### *Suitable excipients and/or diluents*

According to a preferred form, the nanoemulsifying composition also comprises suitable excipients and/or diluents.

Preferably, for the purposes of the invention, suitable excipients and/or diluents are selected from the group consisting of: at least one viscosity inducing agent; at least one preservative; at least one sweetener; at least one antioxidant and/or stabilising agent; at least one acidifying agent; glycerol; and mixtures thereof.

Preferably, the at least one viscosity inducing agent; the at least one preservative; the at least one sweetener; the at least one antioxidant and/or stabilising agent; glycerol are dissolved in the aqueous phase.

Preferably, the glycerol is in an amount comprised between 2% and 10% by weight, preferably between 3% and 10% by weight, preferably between 5% and 9% by weight, preferably between 5% and 8% by weight, preferably about 7.8% by weight, or 5% by weight, of the total volume of the composition.

The viscosity inducing agent is preferably a polymer selected from the group consisting of: sodium hyaluronate, methylcellulose, hydroxyethylcellulose, hydroxypropyl methylcellulose, chitosan.

Preferably, the sodium hyaluronate has medium and/or high molecular weight (between 750 and 2000 KD).

Preferably, the chitosan is highly deacetylated, preferably in a cationised form.

Preferably, the at least one viscosity inducing agent is in an amount comprised between 0.03% and 0.1% by weight, preferably between 0.05% and 0.1% by weight, preferably between 0.07% and 0.1% by weight, preferably between 0.08% and 0.1% by weight, of the total volume of the composition.

Preferably, the at least one sweetening agent is selected from the group consisting of sucralose, acesulfame, aspartame, rebaudoside, polyols, and mixtures thereof.

Preferably, the at least one antioxidant and/or stabilising agent is tocopherol.

Preferably, the at least one preservative is selected from sodium benzoate and potassium sorbate.

Preferably, the nanoemulsifying composition comprises at least one acidifying agent; preferably the latter is selected from: citric acid, tartaric acid, malic acid, and mixtures thereof. Preferably, the acidifying agent is added following the formation of the nanoemulsion.

It should be noted that the nanoemulsifying composition has a $pH < 6$, preferably it has an acidic pH, preferably it has a $pH \leq 4.5$, preferably it has a $pH \leq 4.2$, preferably it has a pH comprised between 3.5 and 4.2, preferably the pH is equal to 4.2. In this sense, the nanoemulsifying composition is compatible with acidic pH values, even below 4.

The acidic environment is mainly generated by the presence of the at least one acidifying agent; the acidic environment contributes to the further stabilisation of the nanoemulsifying composition of the invention, as well as facilitating the activation of the aforesaid preservative.

### *Use of the nanoemulsifying composition*

Preferably, the nanoemulsifying composition is for use in the treatment of vitamin deficiency states, preferably for use in the treatment of states of altered gastro-intestinal assimilation of vitamins $K_2$ and/or $D_3$ and/or $B_{12}$.

For example, vitamin deficiency states or states of altered gastro-intestinal assimilation may be caused by or occur concomitantly with a condition selected from the group consisting of: enteric malabsorption syndrome; impaired biliary secretion; chronic drug use (e.g., long-term therapy with Proton Pump Inhibitors (PPIs), which reduce, to the point of nullifying, the secretion of Castle Intrinsic Factor (IF) by the gastric mucosal cells); malnutrition or inadequate diet; supplemental diet post bariatric surgery; atrophic gastritis; hepatobiliary insufficiency; and mixtures of the above.

Preferably, the nanoemulsifying composition is for use in the treatment of vitamin deficiency states or states of altered gastro-intestinal assimilation of vitamins $K_2$ and/or $D_3$ and/or $B_{12}$ in subjects of senile age, preferably subjects aged $\geq 60$ years.

Preferably, the nanoemulsifying composition is for sublingual use.

Preferably, the nanoemulsifying composition is in the form of a food supplement, medicinal speciality, food for special medical purposes.

A food supplement means a formulation that falls under the definition underlying Directive 2002/46/EC as amended. In this regulation, food supplements are defined as: "food products intended to supplement the common diet and constituting a concentrated source of nutrients, such as vitamins and minerals, or other substances having a nutritional or physiological effect, in particular, but not exclusively, amino acids, essential fatty acids, fibre and extracts of plant origin, both mono- and multi- compound, in pre-dosed forms".

Medicinal product means a pre-packaged pharmaceutical form, industrially manufactured and authorised on the basis of a report containing the chemical, biological, pharmaceutical, pharmaco-toxicological and clinical experimental results relating to the drug, which is marketed under a special name (registered trademark).

According to Regulation (EU) 609/2013, a Food for Special Medical Purposes (FSMP) is defined as a "food specially processed or formulated and intended for the dietary management of patients, including infants, to be used under medical supervision; it is intended for the exclusive or partial feeding of patients with a limited, impaired or disturbed capacity to take, digest, absorb, metabolise or excrete ordinary food or certain nutrients contained therein, or metabolites, or with other medically-determined nutrient requirements, whose dietary management cannot be achieved by modification of the normal diet alone".

### Process for preparing the carrier and nanoemulsifying composition

The preparation process of the *carrier* comprised in the nanoemulsifying composition of the invention comprises the following steps:

a') preparing the aqueous phase and the oil phase in parallel;

b') heating the aqueous phase and the oil phase in parallel to a temperature $\leq 50°C$, preferably comprised between 40°C and 45°C;

c') under mechanical stirring, preferably by stirring for at least 10 minutes, combining the aqueous phase and oil phases at a temperature $\leq 50°C$, preferably comprised between 40°C and 45°C, preferably obtaining a nanoemulsion.

Note that the fat phase is poured in a drizzle onto the aqueous phase under mechanical stirring, preferably at a rotation speed comprised between 750 and 1000 rpm and for a time of at least 5 minutes, preferably for a time comprised between 5 and 45 minutes, preferably between 10 and 45 minutes, preferably between 15 and 45 minutes, preferably between 15 and 30 minutes.

Preferably, the process for preparing the nanoemulsifying composition comprises the following steps:

a) preparing the aqueous phase and the oil phase in parallel;
b) optionally, adding fat-soluble vitamin(s), preferably vitamin $D_3$ and/or vitamin $K_2$, in the oil phase;
b) heating the aqueous phase and the oil phase in parallel to a temperature $\leq 50°C$, preferably comprised between 40°C and 45°C;
c) under mechanical stirring, preferably by stirring for at least 10 minutes, combining the aqueous phase and oil phases at a temperature $\leq 50°C$, preferably comprised between 40°C and 45°C, preferably obtaining a nanoemulsion;
e) optionally, once room temperature has been reached, preferably a temperature of 25°C, adding the water-soluble vitamin, preferably vitamin $B_{12}$, under mechanical stirring, preferably stirring for at least 10 minutes.

EXAMPLES

Below, the Applicant sets out examples for illustrative but not limiting purposes.

### Example 1 - Vitamin $D_3$ carrying nanoemulsifying composition

| Phase | Component | g/ 100 ml | g/0.19 ml | g/20 ml |
|-------|-----------|-----------|-----------|---------|
| A | Polysorbate 80 | 4.0000 | 0.007600 | 0.800 |
| A | Vitamin D$_3$ (40 mil IU/g) | 0.0165 | 0.000031 | 0.003 |
| A | Labrafac (MTC) | 0.8350 | 0.001587 | 0.167 |
| B | Glycerol | 8.0000 | 0.015200 | 1.600 |
| B | Sodium Hyaluronate | 0.0800 | 0.000152 | 0.016 |
| B | Cleargum CRO (Octenyl Succinate Starch) | 1.0000 | 0.001900 | 0.200 |
| B | Demi water | 87.8635 | 0.166941 | 17.573 |
| C | Citric acid | q.s. pH=4.2 | q.s. pH=4.2 | q.s. pH=4.2 |
| B | Potassium sorbate | 0.1000 | 0.000190 | 0.020 |
| D | Liquid vanilla flavouring | 0.1000 | 0.000190 | 0.020 |
| B | Sucralose | 0.0050 | 0.000010 | 0.001 |
| | | **102.00** | **0.193800** | **20.400** |

*Preparation process:*

Phase B: first disperse the Glycerine and then the Sodium Hyaluronate at room temperature under vigorous stirring until completely gelled and free of lumps. Add the Cleargum, potassium sorbate and sucralose to the clear system under constant stirring until a clear phase is obtained.

Phase A: weigh the Labrafac and proceed to disperse the Vitamin D$_3$ into the Labrafac. Add the Polysorbate 80 and stir until clear and homogeneous.

Heat Phase A and Phase B to a temperature comprised between 40 and 45°C. Combine Phase A with Phase B under vigorous stirring and continue stirring for 10 minutes. Finally, adjust the pH to 4.2 with citric acid (Phase C) and add flavouring (Phase D).

***Example 2** - **Vitamin K$_2$ carrying nanoemulsifying composition***

| Phase | Component | g/ 100 ml | g/0.19 ml | g/20 ml |
|---|---|---|---|---|
| A | Polysorbate 80 | 6.0000 | 0.011400 | 1.200 |
| A | Vitamin $K_2$ (10,000 ppm in MCT) | 2.7000 | 0.005130 | 0.540 |
| B | Glycerol | 8.0000 | 0.015200 | 1.600 |
| B | Sodium Hyaluronate | 0.0800 | 0.000152 | 0.016 |
| B | Cleargum CRO (Octenyl Succinate Starch) | 2.0000 | 0.003800 | 0.400 |
| B | Demi water | q.s. 100 ml | q.s. to 0.19 ml | q.s. to 20 ml |
| C | Citric acid | q.s. pH=4.2 | q.s. pH=4.2 | q.s. pH=4.2 |
| B | Potassium sorbate | 0.1000 | 0.000190 | 0.020 |
| D | Liquid vanilla flavouring | 0.1000 | 0.000190 | 0.020 |
| B | Sucralose | 0.0050 | 0.000009 | 0.001 |
| | | **102.00** | **0.190000** | **20.400** |

*Preparation process:*

Phase A: proceed to disperse the Vitamin $K_2$ in MCT in Polysorbate 80 and mix until clear and homogeneous.

Prepare Phase B by dispersing first the Glycerine and then the Sodium Hyaluronate at room temperature under vigorous stirring until completely gelled and free of lumps. Add the Cleargum, potassium sorbate and sucralose to the clear system under constant stirring until a clear phase is obtained.

Heat Phase A and Phase B to a temperature comprised between 40 and 45°C and combine Phase A with Phase B under vigorous stirring while maintaining the temperature. Bring the system to a temperature of 25°C and adjust the pH to 4.2 with citric acid (Phase C). Add the flavouring (Phase D).

**Example 3** - *Vitamin $B_{12}$ carrying nanoemulsifying composition*

| Phase | Component | g/ 100 ml | g/0.19 ml | g/20 ml |
|-------|-----------|-----------|-----------|---------|
| C | Vitamin B$_{12}$ (Methylcobalamin) | 0.3158 | 0.00060 | 0.063 |
| B | Cleargum CRO 01 (Octenyl Succinate Starch) | 1.0000 | 0.00190 | 0.200 |
| B | Glycerol | 5.0000 | 0.00095 | 1.00 |
| B | Sodium Hyaluronate | 0.0800 | 0.000152 | 0.016 |
| A | Polysorbate 80 | 1.0000 | 0.00190 | 0.200 |
| B | Demi water | q.s. 100 ml | q.s. to 0.19 ml | q.s. to 20 ml |
| A | MCT (Caprylic/Capric Triglyceride) | 3.0000 | 0.00570 | 0.30000 |
| D | Citric acid | q.s. pH=4.2 | q.s. pH=4.2 | q.s. pH=4.2 |
| A | Potassium sorbate | 0.1000 | 0.00019 | 0.020 |
| E | Berry flavouring | 0.2000 | 0.00038 | 0.040 |
| A | Sucralose | 0.0050 | 0.0000095 | 0.001 |
| | | **102.00** | **0.190000** | **20.40** |

*Preparation process:*

Phase A: Disperse Labrafac (MCT (Caprylic/Capric Triglyceride)) in Polysorbate 80 and mix until clear and homogeneous.

Phase B: first disperse the Glycerine and then the Sodium Hyaluronate at room temperature under vigorous stirring until completely gelled and free of lumps. Add the Cleargum, potassium sorbate and sucralose to the clear system under constant stirring until a clear phase is obtained.

Heat Phase A and Phase B to a temperature comprised between 40 and 45°C and combine Phase A with Phase B under vigorous stirring while maintaining the temperature. Continue stirring for 10 minutes. Bring the system to a temperature of 25°C and proceed to disperse the Vitamin B12 (Phase C) continuing to stir for 10 minutes. Next, adjust the pH to 4.2 with citric acid (Phase D) and then add the flavouring (Phase E).

**Example 4** - *Nanoemulsifying composition delivering vitamin D$_3$, vitamin K$_2$ and vitamin B$_{12}$*

| Phase | Component | g/ 100 ml | g/0.19 ml | g/20 ml |
|-------|-----------|-----------|-----------|---------|
| A | Polysorbate 80 | 6.0000 | 0.011400 | 1.200 |
| A | Vitamin $K_2$ (10,000 ppm in MCT) | 2.7000 | 0.005130 | 0.540 |
| A | Vitamin $D_3$ (40 mil IU/g) | 0.0165 | 0.000031 | 0.003 |
| B | Glycerol | 8.0000 | 0.015200 | 1.600 |
| B | Sodium Hyaluronate | 0.0800 | 0.000152 | 0.016 |
| B | Cleargum CRO (Octenyl Succinate Starch) | 2.0000 | 0.003800 | 0.400 |
| B | Demi water | q.s. to 100 ml | q.s. to 0.19 ml | q.s. to 20 ml |
| C | Vitamin $B_{12}$ (Methylcobalamin) | 0.3158 | 0.00060 | 0.063 |
| D | Citric acid | q.s. pH=4.2 | q.s. pH=4.2 | q.s. pH=4.2 |
| B | Potassium sorbate | 0.1000 | 0.000190 | 0.020 |
| E | Liquid vanilla flavouring | 0.1000 | 0.000190 | 0.020 |
| B | Sucralose | 0.0050 | 0.000009 | 0.001 |
| | | **102.00** | **0.190000** | **20.400** |

*Preparation process:*

Phase A: proceed to combine Polysorbate 80, Vitamin $K_2$ in MCT, and Vitamin $D_3$ while mixing until clear and homogeneous.

Phase B: first disperse the Glycerine and then the Sodium Hyaluronate at room temperature under vigorous stirring until completely gelled and free of lumps. Add the Cleargum, potassium sorbate and sucralose to the clear system under constant stirring until a clear phase is obtained.

Heat Phase A and Phase B to a temperature comprised between 40 and 45°C and combine Phase A with Phase B under vigorous stirring while maintaining the temperature. Continue stirring for 10 minutes. Bring the system to a temperature of 25°C and proceed to disperse the Vitamin $B_{12}$ (Phase C) continuing to stir for 10 minutes. Next, adjust the pH to 4.2 with citric acid (Phase D) and then add the flavouring (Phase E).

***Example 5 - Comparative laboratory test***

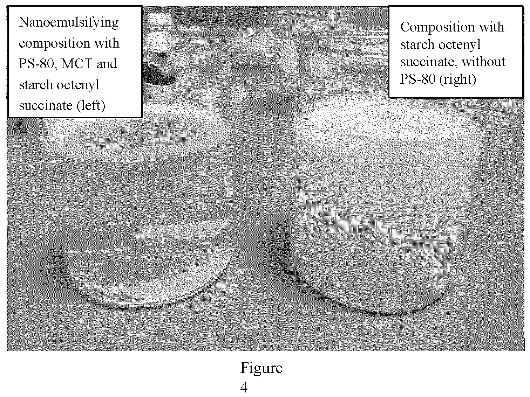

Figure 4 shows two beakers, of which:

- the beaker on the left contains the nanoemulsifying composition of the invention, containing vitamin $D_3$, the at least one polyoxyethylene sorbitan ester, namely polysorbate 80, a medium chain triglyceride, namely Caprylic/Capric Triglyceride, and modified alkyl carboxylate starch, namely starch sodium octenyl succinate;

- on the other hand, the beaker on the right contains the comparative or control composition (not in line with the invention) characterised by the same essential components as the nanoemulsifying composition of the invention in the beaker on the left, but *devoid* of polysorbate 80.

The nanoemulsifying composition of the left beaker appears completely transparent, indicating an extremely small dispersed phase droplet size (*back scattering* phenomenon), unlike the control composition of the right beaker.

The nanoemulsifying composition of the beaker on the left in accordance with the invention remains clear and transparent after 60 days at 40°C without any significant pH changes or organoleptic changes worthy of note.

This demonstrates that, in order to obtain a transparent nanoemulsion, the presence of the emulsifying pair of at least one polyoxyethylene sorbitan ester and modified alkyl carboxylate starch is essential. The mere presence of modified alkyl carboxylate starch, however, did not prove sufficient for the above-mentioned purposes.

Note that the same test was also performed in parallel for vitamin $K_2$ and vitamin $B_{12}$, obtaining the same positive results as described above.

*Example 6 - DLS Analysis*

DLS (*Dynamic Light Scattering*) analysis was performed for the nanoemulsifying composition of the invention, comprising vitamin $D_3$, to verify its actual nanometric size.

*Details of the sample analysed:*

| RI (*Refractive Index*) of the material | 1.59 |
|---|---|
| Material absorption | 0.010 |
| Dispersing agent | Water |
| RI (*Refractive Index*) of the dispersant | 1,330 |
| Viscosity (cP) | 0.8872 |

*Details of system settings:*

| Temperature (°C) | 25 |
|---|---|
| Counting speed (kcps) | 192.3 |
| Cell description | Disposable calibration cuvette |
| Duration used (s) | 70 |
| Measuring position (mm) | 0.85 |
| Attenuator | 7 |

*Results:*

| Average-Z (d.nm) | 55.28 |
|---|---|
| PdI (*Polydispersity Index*) | 0,365 |
| Interception | 0,843 |
| Quality of the result | Good |

|  | Size (d.nm) | % Intensity | Std. Dev. (d.nm) |
|---|---|---|---|
| Peak 1 | 62.33 | 86.8 | 34.25 |
| Peak 2 | 2736 | 13.2 | 1306 |
| Peak 3 | 0,000 | 0.0 | 0,000 |

**REFERENCES**

1. Bayraktar N, Turan H, Bayraktar M, Ozturk A, Erdo ğ du H. Analysis of serum cytokine and protective vitamin D levels in severe cases of COVID-19. J Med Virol. 2022 Jan;94(1):154-160. doi: 10.1002/jmv.27294.

2. Wang J, Yang X, Li Y, Huang JA, Jiang J, Su N. Specific cytokines in the inflammatory cytokine storm of patients with COVID-19-associated acute respiratory distress syndrome and extrapulmonary multiple-organ dysfunction. Virol J. 2021 Jun 4;18(1):117. doi: 10.1186/s12985-021-01588-y.

3. Harrison SR, Li D, Jeffery LE, Raza K, Hewison M. Vitamin D, Autoimmune Disease and Rheumatoid Arthritis. Calcif Tissue Int. 2020 Jan;106(1):58-75. doi: 10.1007/s00223-019-00577-2.

4. Myneni VD, Mezey E. Regulation of bone remodeling by vitamin K2. Oral Dis. 2017 Nov;23(8):1021-1028. doi: 10.1111/odi.12624.

5. Cockayne S, Adamson J, Lanham-New S, Shearer MJ, Gilbody S, Torgerson DJ. Vitamin K and the prevention of fractures: systematic review and meta-analysis of randomized controlled trials. Arch Intern Med. 2006 Jun 26;166(12):1256-61. doi: 10.1001/archinte.166.12.1256.

6. Hariri E, Kassis N, Iskandar J, et al Vitamin K2-a neglected player in cardiovascular health: a narrative review Open Heart 2021;8:e001715. doi: 10.1136/openhrt-2021-001715.

7. Reboul E Intestinal absorption of vitamin D: from the meal to the enterocyte. Food Funct. 2015;6(2):356-62. DOI: 10.1039/c4fo00579a.

8. M Rautureau and J C Rambaud. Aqueous solubilisation of vitamin D3 in normal man. Gut. 1981; 22(5): 393-397. DOI: 10.1136/gut.22.5.393.

9. Satia MC, Mukim AG, Tibrewala KD, Bhavsar MS. A randomized two way cross over study for comparison of absorption of vitamin D3 buccal spray and soft gelatin capsule formulation in healthy subjects and in patients with intestinal malabsorption. Nutr J. 2015 29;14:114. DOI: 10.1186/s12937-015-0105-1.

10. Cristian Del Bo', Patrizia Riso, Claudio Gardana, Antonella Brusamolino, Alberto Battezzati, Salvatore Ciappellano. Effect of two different sublingual dosages of vitamin B12 on cobalamin nutritional status in vegans and vegetarians with a marginal deficiency: A randomized controlled trial, Clinical Nutrition, Volume 38, Issue 2, 2019,Pages 575-583,ISSN 0261-5614, https://doi.org/10.1016/j.clnu.2018.02.00.

11. Tu ğ ba-Kartal A, Ça ğ la-Mutlu Z. Comparison of Sublingual and Intramuscular Administration of Vitamin B12 for the Treatment of Vitamin B12 Deficiency in Children. Rev Invest Clin. 2020 Dec 22;72(6):380-385. doi: 10.24875/RIC.20000208. PMID: 33053572.

12. Fratter A, Semenzato A. New association of surfactants for the production of food and cosmetic nanoemulsions: preliminary development and characterization. Int J Cosmet Sci. 2011 Oct;33(5):443-9. doi: 10.1111/j. 1468-2494.2011.00652.x.

13. Fratter A, Biagi D, Cicero AFG. Sublingual Delivery of Astaxanthin through a Novel Ascorbyl Palmitate-Based Nanoemulsion: Preliminary Data. Mar Drugs. 2019 Aug 29;17(9):508. doi: 10.3390/md17090508.

**Claims**

1. Nanoemulsifying composition comprising

   a carrier comprising or consisting of

       i) an oil phase containing

           - at least one polyoxyethylene sorbitan ester,
           - a medium-chain triglyceride,

       ii) an aqueous phase containing

           - a modified alkyl carboxylate starch,
           - water,

   an active ingredient, dissolved or dispersed in the aqueous phase and/or in the oil phase, said active ingredient consisting of a vitamin selected from the group consisting of Vitamin $D_3$, Vitamin $K_2$, Vitamin $B_{12}$, and mixtures thereof,
   wherein the composition does not comprise ascorbic acid and/or ascorbyl-conjugated fatty acids, preferably it does not comprise ascorbyl palmitate.

2. Composition according to claim 1, wherein the at least one polyoxyethylene sorbitan ester is selected from the group consisting of: polysorbate 20, polysorbate 40, polysorbate 60, polysorbate 65, polysorbate 80, and mixtures thereof.

3. Composition according to claim 1 or 2, wherein the at least one polyoxyethylene sorbitan ester is in an amount between 0.3% and 10% by weight of the total volume of the composition.

4. Composition according to any one of claims 1 to 3, wherein the medium-chain triglyceride is in an amount between 0.5% and 5% by weight of the total volume of the composition.

5. Composition according to any one of claims 1 to 4, wherein the modified alkyl carboxylate starch is selected from: sodium starch octenyl succinate and aluminate starch octenyl succinate.

6. Composition according to any one of claims 1 to 5, wherein the modified alkyl carboxylate starch is in an amount between 0.3% and 5% by weight of the total volume of the composition.

7. Composition according to any one of claims 1 to 6, wherein water is in an amount between 75% and 95% by weight of the total volume of the composition.

8. Composition according to any one of claims 1 to 7, wherein the average diameter size of the dispersed phase particles is between 50 and 200 nm.

9. Composition according to any one of claims 1 to 8, in the form of food supplement, food for special medical purposes, medicinal product.

10. Composition according to any one of claims 1 to 9, for sublingual use.

11. Composition according to any one of claims 1 to 10, for use in the treatment of states of impaired gastro-intestinal assimilation of vitamin $D_3$ and/or vitamin $K_2$ and/or vitamin $B_{12}$.

12. Process for preparing the composition according to any one of claims 1 to 11, comprising the following steps:

   a) preparing the aqueous phase and the oil phase in parallel;
   b) optionally, adding fat-soluble vitamin(s), preferably vitamin $D_3$ and/or vitamin $K_2$, in the oil phase;

c) heating the aqueous phase and the oil phase in parallel to a temperature ≤ 50°C;

d) under mechanical stirring, combining the aqueous phase and oil phases at a temperature ≤ 50°C;

e) optionally, once reached room temperature, adding the water-soluble vitamin under mechanical stirring.

Figure
1

Figure
2

Figure 3

Nanoemulsifying composition with PS-80, MCT and starch octenyl succinate (left)

Composition with starch octenyl succinate, without PS-80 (right)

Figure
4

Size distribution by intensity

Figure
5

Europäisches
Patentamt

European
Patent Office

Office européen
des brevets

# EUROPEAN SEARCH REPORT

Application Number

EP 24 19 7910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/024126 A2 (KARNAK TECH LLC [US]; EZRA RAFAEL [IL]) 3 February 2022 (2022-02-03) * pages 50, 68 * * examples 1,3,10 * * claim 25 * * paragraph [04.3] * ----- | 1-12 | INV. A61K9/51 A61K9/107 A61K47/10 A61K47/36 |
| A | US 11 351 117 B2 (NULIXIR INC [US]) 7 June 2022 (2022-06-07) * claim 1 * ----- | 1-12 | |

TECHNICAL FIELDS
SEARCHED      (IPC)

A61K

The present search report has been drawn up for all claims

| Place of search | Date of completion of the search | Examiner |
|---|---|---|
| The Hague | 23 December 2024 | Friederich, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 7910

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2024

| Patent document cited in search report | Publication date | Patent family member(s) | | Publication date |
|---|---|---|---|---|
| WO 2022024126 A2 | 03-02-2022 | AU | 2021317262 A1 | 09-06-2022 |
| | | CA | 3166084 A1 | 03-02-2022 |
| | | CN | 115843242 A | 24-03-2023 |
| | | EP | 4057993 A2 | 21-09-2022 |
| | | IL | 293086 A | 01-07-2022 |
| | | KR | 20220085816 A | 22-06-2022 |
| | | US | 2023345992 A1 | 02-11-2023 |
| | | WO | 2022024126 A2 | 03-02-2022 |
| US 11351117 B2 | 07-06-2022 | BR | 112022004419 A2 | 31-05-2022 |
| | | CA | 3150979 A1 | 18-03-2021 |
| | | CN | 115243568 A | 25-10-2022 |
| | | CN | 117598506 A | 27-02-2024 |
| | | EP | 4009815 A2 | 15-06-2022 |
| | | US | 2021077394 A1 | 18-03-2021 |
| | | US | 2021077411 A1 | 18-03-2021 |
| | | US | 2021154139 A1 | 27-05-2021 |
| | | US | 2021169816 A1 | 10-06-2021 |
| | | US | 2021169820 A1 | 10-06-2021 |
| | | US | 2021236433 A1 | 05-08-2021 |
| | | US | 2021275461 A1 | 09-09-2021 |
| | | US | 2022110867 A1 | 14-04-2022 |
| | | US | 2022211619 A1 | 07-07-2022 |
| | | US | 2022331246 A1 | 20-10-2022 |
| | | US | 2024382421 A1 | 21-11-2024 |
| | | WO | 2021051105 A2 | 18-03-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011073726 A1

### Non-patent literature cited in the description

- **WANG J** ; **YANG X** ; **LI Y** ; **HUANG JA** ; **JIANG J** ; **SU N**. Specific cytokines in the inflammatory cytokine storm of patients with COVID-19-associated acute respiratory distress syndrome and extrapulmonary multiple-organ dysfunction. *Virol J.*, 04 June 2021, vol. 18 (1), 117
- **HARRISON SR** ; **LI D** ; **JEFFERY LE** ; **RAZA K** ; **HEWISON M** ; **VITAMIN D**. Autoimmune Disease and Rheumatoid Arthritis. *Calcif Tissue Int.*, January 2020, vol. 106 (1), 58-75
- **MYNENI VD** ; **MEZEY E**. Regulation of bone remodeling by vitamin K2. *Oral Dis.*, November 2017, vol. 23 (8), 1021-1028
- **COCKAYNE S** ; **ADAMSON J** ; **LANHAM-NEW S** ; **SHEARER MJ** ; **GILBODY S** ; **TORGERSON DJ**. Vitamin K and the prevention of fractures: systematic review and meta-analysis of randomized controlled trials. *Arch Intern Med.*, 26 June 2006, vol. 166 (12), 1256-61
- **HARIRI E** ; **KASSIS N** ; **ISKANDAR J et al.** Vitamin K2-a neglected player in cardiovascular health: a narrative review. *Open Heart*, 2021, vol. 8, e001715
- **REBOUL E**. Intestinal absorption of vitamin D: from the meal to the enterocyte. *Food Funct.*, 2015, vol. 6 (2), 356-62

- **M RAUTUREAU** ; **J C RAMBAUD**. Aqueous solubilisation of vitamin D3 in normal man. *Gut.*, 1981, vol. 22 (5), 393-397
- **SATIA MC** ; **MUKIM AG** ; **TIBREWALA KD** ; **BHAVSAR MS**. A randomized two way cross over study for comparison of absorption of vitamin D3 buccal spray and soft gelatin capsule formulation in healthy subjects and in patients with intestinal malabsorption. *Nutr J.*, 2015, vol. 29 (14), 114
- **CRISTIAN DEL BO** ; **PATRIZIA RISO** ; **CLAUDIO GARDANA** ; **ANTONELLA BRUSAMOLINO** ; **ALBERTO BATTEZZATI** ; **SALVATORE CIAPPELLANO**. Effect of two different sublingual dosages of vitamin B12 on cobalamin nutritional status in vegans and vegetarians with a marginal deficiency: A randomized controlled trial. *Clinical Nutrition*, 2019, vol. 38 (2), ISSN 0261-5614, 575-583, https://doi.org/10.1016/j.clnu.2018.02.00
- **FRATTER A** ; **SEMENZATO A**. New association of surfactants for the production of food and cosmetic nanoemulsions: preliminary development and characterization.. *Int J Cosmet Sci.*, October 2011, vol. 33 (5), 443-9
- **FRATTER A** ; **BIAGI D** ; **CICERO AFG**. Sublingual Delivery of Astaxanthin through a Novel Ascorbyl Palmitate-Based Nanoemulsion: Preliminary Data. *Mar Drugs.*, 29 August 2019, vol. 17 (9), 508